# EUROPÄISCHE PATENTANMELDUNG

(11) **EP 1 142 530 A1**
(43) Veröffentlichungstag der Anmeldung: **10.10.2001**
(21) Anmeldenummer: 01106906.9
(22) Anmeldetag: 20.03.2001
(51) Int. Cl.: A61B 1/273, A61B 8/12, A61M 25/10, A61M 25/01

(54) **Pneumatische Steuerungsvorrichtung für ein Endoskop**

(30) Priorität: 21.03.2000 DE 10013685
(71) Anmelder: Zotz, Rainer, Dr., 65203 Wiesbaden (DE)
(72) Erfinder: Zotz, Rainer, Dr., 65203 Wiesbaden (DE)
(74) Vertreter: Ahrens, Gabriele, Dr.

(57) **Zusammenfassung**

Die vorliegende Erfindung betrifft eine Vorrichtung zur pneumatischen Steuerung eines Endoskops, wobei die Vorrichtung als Manschette ausgebildet ist, die um das Endoskop gelegt wird, und die Manschette mindestens eine Kammer zur Aufnahme eines Fluids sowie eine Zufuhr für das Fluid aufweist.

## Beschreibung

Die vorliegende Erfindung betrifft eine pneumatische Steuerungsvorrichtung für ein Endoskop wie es für Ultraschalluntersuchungen eingesetzt wird.

Als nichtinvasives Abbildungsverfahren findet das Ultraschallverfahren in der Medizin zur Diagnose breite Anwendung, da auf vergleichsweise komplikationslose Weise ohne operativen Eingriff Aufschluss über krankhafte Veränderungen eines Organismusses erhalten werden können.

Da das Ultraschallverfahren eine Schnittbildmethode ist, das je nach Stellung des Schallkopfes lediglich eine Ebene wiedergibt, muss der Schallkopf zur vollständigen Darstellung über den interessierenden Bereich bewegt werden können.

Ein wichtiges Anwendungsgebiet für die Ultraschalldiagnostik ist die transösophageale Echokardiographie, bei der der Schallkopf an der Spitze eines flexiblen Endoskop angebracht ist und über die Speiseröhre in unmittelbare Nähe des Herzens vorgeschoben werden kann.

Um eine möglichst vollständige Abbildung des Herzens und seiner Umgebung, insbesondere der Herzkranzgefäße, erhalten zu können, muss der Schallkopf in möglichst viele unterschiedliche Positionen gebracht werden können.
Dies geschieht derzeit durch manuelle Steuerung am Anfang des Endoskops oder durch elektronische Verschiebung. Bei der elektronischen Steuerung ist jedoch von Nachteil, dass derzeit die Rotation nur in einer Ebene möglich ist.

So ist bei der heutzutage eingesetzten multiplanen transösophagealen Echiokardiographie (multiplane TEE) der Schallkopf senkrecht an der Seite des Endoskops angeordnet und rotiert in dieser senkrechten X-Achse. Obwohl mit den bekannten Steuerungsmechanismen weite Teile des Herzens erfasst werden können, hat sich gezeigt, dass diese vorhandenen Steuerungsmöglichkeiten für bestimmte Bereiche nur ungenügend sind. So lassen sich damit beispielsweise die linke Herzkranzschlagader (Riva) und die rechte Kranzarterie nicht reproduzierbar erfassen.

Es bestand daher ein Bedarf an weiteren Steuerungsmöglichkeiten, um den Schallkopf in zusätzliche Ebenen bringen zu können, und damit auch Bereiche zugänglich zu machen, die bisher nicht oder nur ungenügend erfasst werden.

Erfindungsgemäß wird diese Aufgabe durch eine Vorrichtung zur pneumatischen Steuerung für ein Endoskop gelöst, dadurch gekennzeichnet, dass die Vorrichtung als Manschette ausgebildet ist, die um das Endoskop gelegt ist, wobei die Manschette mindestens eine Kammer zur Aufnahme eines Fluids sowie eine Zufuhr für das Fluid aufweist.

Wird nun die Kammer mit dem Fluid beschickt, dehnt sich die Kammer aus und übt Druck auf das Endoskop bzw. dessen Umgebung aus, wodurch sich die Position des Endoskops bzw. des Schallkopfes ändert.

Die Manschette kann eine oder mehrere Kammern enthalten. Die Kammern können getrennt sein, alle oder einzelne Kammern können miteinander verbunden sein, so dass ein Fluidfluss zwischen den verbundenen Kammern möglich ist.

Die Kammer bzw. Kammern können einen Ring bilden, der den Endoskopkörper umfasst oder kappenartig zur Aufnahme der Endoskopspitze ausgestaltet sein.
Die Vorrichtung kann aus mehreren Ringen bestehen, die optional mit der kappenartigen Ausgestaltungsform kombiniert werden können.

Je nach Position der Manschette an dem Endoskop, Anzahl und Anordnung der Kammern kann je nach Menge Fluid, die einer Kammer zugeführt wird, der Druck, der von den einzelnen Kammern ausgeübt wird, gesteuert werden und damit der Schallkopf in entsprechend unterschiedliche Positionen gebracht werden.

Im einfachsten Fall wird durch die Druckausübung der Abstand des Schallkopfes zum Herzen vergrößert, wodurch sich auch der Schallwinkel vergrößert und eine entsprechend erweiterte Abbildung ermöglicht.

Dieser Vorgang der Vergrößerung des Abstandes wird üblicherweise als "Einbringen einer Vorlaufstrecke" bezeichnet.

Das Fluid wird über eine Leitung in die Kammern eingebracht, wobei voneinander getrennte Kammern jeweils eine separate Zuleitung aufweisen.

Für die Wirkweise der erfindungsgemäßen Vorrichtung ist die Art des Fluids nicht kritisch. Da das Endoskop mit der Manschette bei der hier beschriebenen Anwendung in die Speiseröhre eingeführt wird, sollte das Fluid medizinisch unbedenklich sein, zudem sollte es den Ultraschall nicht behindern.

Ein Fluid, das die oben genannten Voraussetzungen erfüllt, ist z. B. Wasser.

Das Material für die Manschette muss sich bei Beschickung mit dem Fluid ausreichend dehnen können, um den gewünschten Druck ausüben zu können, d. h. das Material muss eine ausreichende Elastizität (Compliance) aufweisen, vorzugsweise sollte es sich bei Ablassen des Fluids wieder zusammenziehen können, und es muss eine genügende Festigkeit haben, damit sichergestellt ist, dass die Manschette nicht reißt
Darüber hinaus sollte das Material medizinisch unbedenklich sein.

Die Anforderungen an die Elastizität und die Festigkeit können dabei je nach konkretem Anwendungsfall verschieden sein.

Geeignet sind z. B. Kunststoffmaterialien wie sie heutzutage üblicherweise in der Medizintechnik eingesetzt werden, und die die oben genannten Kriterien erfüllen.

Nachstehend wird die erfindungsgemäße Steuerungsvorrichtung anhand der Figuren, die bevorzugte Ausführungsformen der Erfindung wiedergeben, näher erläutert.

Es zeigen:
- **Figur 1**: die verschiedenen Achsen, um die eine TEE-Sonde bewegt werden kann;
- **Figur 2**: den Schallkegel einer TEE-Sonde gemäß Figur 1;
- **Figur 3**: eine bevorzugte Ausgestaltung der erfindungsgemäßen Vorrichtung; und
- **Figur 4**: eine weitere bevorzugte Ausgestaltung der Erfindung.

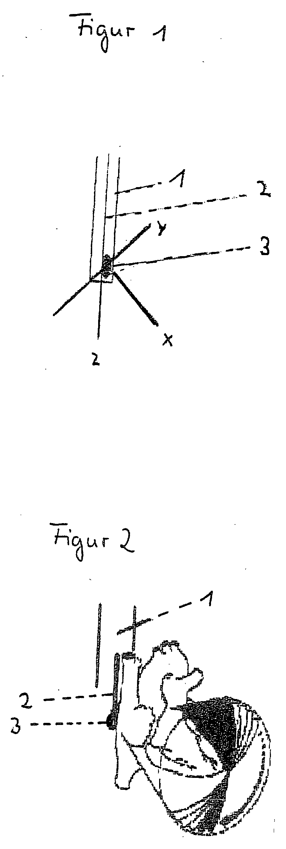

In **Figur 1** sind die möglichen Achsen gezeigt, um die eine TEE-Sonde 2 bewegt werden kann, wobei die z-Achse parallel zur Längsachse der Sonde 2 bzw. der Speiseröhre 1 (Ösophagus) verläuft, die x-Achse die Achse ist, die senkrecht zur z-Achse aus dem Schallkopf 3 herauskommt und um die sich der Schallkopf 3 bei einer 3D-Aufnahme dreht, und die y-Achse rechtwinklig zu den beiden anderen Achsen, d. h. bezogen auf die Abbildung senkrecht zur Blattebene steht.

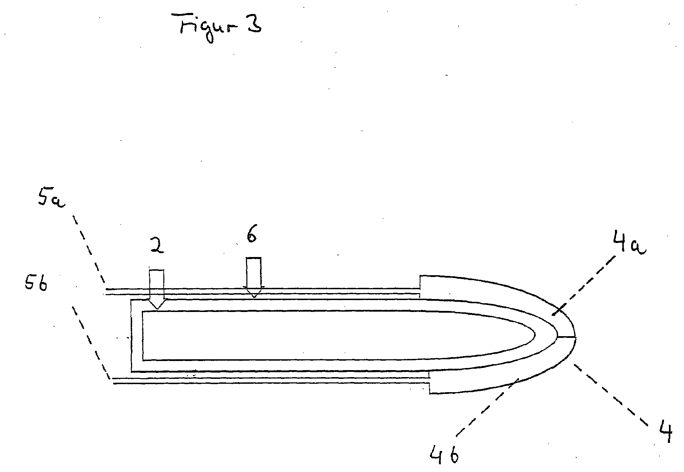

**Figur 2** zeigt den Schallwinkel einer derzeit gängigen transösophagealen Darstellung des Herzens als Gesamtorgan, wobei der Schallkopf 3, der senkrecht zur Seite des Endoskops bzw. Gastroskops 2 angeordnet ist, in dieser senkrechten x-Achse rotiert.

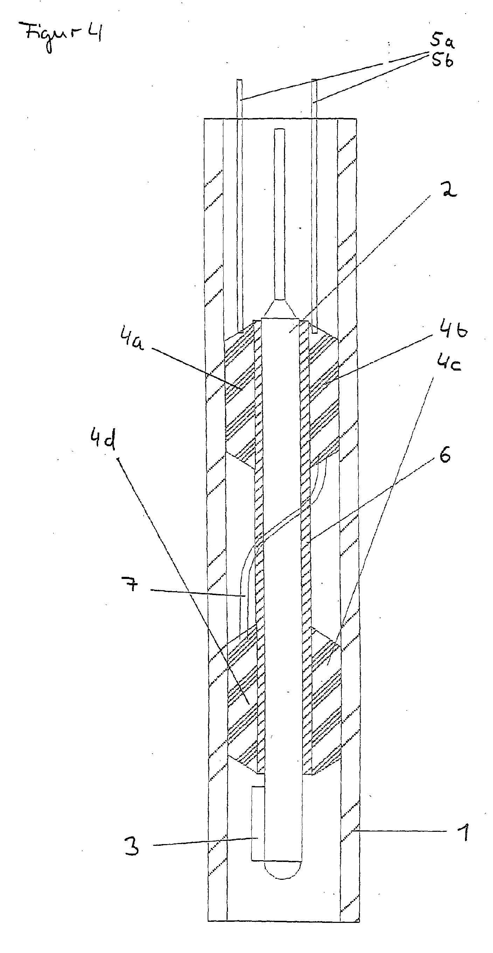

Die in **Figur 3** gezeigte Ausführungsform für eine erfindungsgemäße Vorrichtung 4 zur pneumatischen Steuerung eines Endoskops 2 bzw. Gastroskops umfasst die Spitze des Endoskops 2 kappenartig.

Die gezeigte kappenartig ausgebildete erfindungsgemäße Vorrichtung 4 weist hier zwei getrennte Kammern 4a, 4b zur Aufnahme eines Fluids auf, das über Zuleitungen 5a, 5b den einzelnen Kammern 4a, 4b zugeführt wird.
Über diese Ausgestaltung kann insbesondere die Spitze des Geräts gesteuert werden.

Das Endoskop ist zudem hier mit einem Überzug 6 versehen, wie er üblicherweise aus hygienischen Gründen eingesetzt wird.

Der in Figur 3 gezeigte Abschnitt eines Endoskops hat üblicherweise eine Länge von ca. 50 bis 80 cm und einen Durchmesser von 1 cm.

**Figur 4** zeigt eine weitere Ausgestaltung der Erfindung, bei der die Vorrichtung 4 nicht die Endoskopspitze umgibt sondern ringförmig um den Endoskopkörper angebracht ist.
Jeweils zwei Kammern 4a, 4b und 4c, 4d umfassen unter Ausbildung von zwei Ringen einander gegenüberliegend auf unterschiedlicher Höhe den Endoskopkörper.
Die beiden in der Figur oberen Kammern 4a, 4b verfügen jeweils über eine Zuleitung 5a, 5b zur Zufuhr des Fluids. Die Beschickung der beiden in der Figur unteren Kammern 4c, 4d erfolgt über Verbindungsleitungen 7, wobei hier jeweils die sich auf unterschiedlicher Höhe gegenüberliegenden Kammern 4a, 4c bzw. 4b, 4d miteinander verbunden sind. In Figur 4 ist nur die Verbindung zwischen den Kammern 4b und 4d gezeigt.

In der in Figur 4 gezeigten Ausgestaltung der Erfindung sind die Kammern auf dem Überzug 6 angeordnet und können mit diesem Überzug 6 fest verbunden sein bzw. integraler Bestandteil des Überzugs 6 sein.
Prinzipiell kann die Anzahl der Kammern 4 a, b etc., ihre Größe und ihre Anordnung auf dem Endoskop beliebig je nach Wunsch gewählt werden.

So können die Kammern nur in einer Höhe oder aber auch in mehr als zwei verschiedenen Höhen angeordnet sein, es können gleichzeitig eine oder mehrere Kammern vorgesehen sein, die die Spitze umfassen, wie z. B. in Figur 3 gezeigt.
Die Kammern können alle oder teilweise auf einem Überzug 6 angeordnet sein und/oder mit diesem verbunden sein.

Auch die Art der Fluidzufuhr ist frei je nach Bedarf und Zweck wählbar.
Prinzipiell kann jede Kammer mit einer separaten Zufuhr 5 a, b versehen sein.
Einzelne Kammern können mit einander z. B. über Verbindungsleitungen 7 kommunizieren und über eine einzige gemeinsame Zufuhr verfügen.

Gemäß einer weiteren Ausgestaltung kann die Vorrichtung 4 im Anwendungsfall im gefüllten Zustand einen Ballon ausbilden, der die Endoskopspitze einschließlich Schallkopf 3 umschließt.
Diese Ausgestaltung eignet sich insbesondere für Aufnahmen des Herzens vom Magen aus. Hierzu wird das Endoskop 2 mit Ultraschallkopf 3, das mit der erfindungsgemäßen Vorrichtung 4 versehen ist, in den Magen eingeführt, dort die Vorrichtung 4 mit Fluid gefüllt, wobei sich die das vordere Ende des Endoskops 2 mit Ultraschallkopf 3 umgebende Vorrichtung 4 ballonartig ausdehnt und so auf diese Weise eine entsprechende Vorlaufstrecke eingebracht werden kann.
Die Sondenspitze wird hierbei im Magen so positioniert, dass durch das Zwerchfell hindurch das Herz beschallt wird.
Auch diese Ausgestaltung als Ballon kann in Kombination mit den vorstehend genannten geeigneten Ausgestaltungen eingesetzt werden.

Aufgrund der großen Variationsvielfalt der Ausgestaltung und Anordnung der erfindungsgemäßen Vorrichtung zur pneumatischen Steuerung eines Endoskops kann das Spektrum der Bewegungsmöglichkeiten für derartige Sonden deutlich verbreitert werden.

### Bezugszeichenliste

- 1: Speiseröhre (Ösophagus)
- 2: Endoskop / Gastroskop
- 3: Schallkopf
- 4: Vorrichtung zur pneumatischen Steuerung
- 4a, b, c, d: Kammern
- 5 a, b: Zuleitung für Fluid
- 6: Überzug
- 7: Verbindungsleitung

## Patentansprüche

1. Vorrichtung zur pneumatischen Steuerung eines Endoskops,
**dadurch gekennzeichnet,**
**dass** die Vorrichtung (4) als Manschette ausgebildet ist, die um das Endoskop (2) gelegt ist, wobei die Manschette mindestens eine Kammer (4 a, b, c, d) zur Aufnahme eines Fluids aufweist sowie eine Zufuhr (5 a, b) für das Fluid.

2. Vorrichtung nach Anspruch 1,
**dadurch gekennzeichnet,**
**dass** die Vorrichtung (4) die Endoskopspitze umgibt und sich im gefüllten Zustand ballonartig ausdehnt.

3. Vorrichtung nach Anspruch 1,
**dadurch gekennzeichnet,**
**dass** die Vorrichtung (4) kappenartig zur Aufnahme der Endoskopspitze ausgestaltet ist.

4. Vorrichtung nach Anspruch 1,
**dadurch gekennzeichnet,**
**dass** die Vorrichtung (4) ringförmig ausgestaltet ist.

5. Vorrichtung nach einem der Ansprüche 1, 3 oder 4,
**dadurch gekennzeichnet,**
**dass** die Vorrichtung mindestens zwei voneinander getrennte Kammern (4, a, b, c, d) aufweist.

6. Vorrichtung nach einem der Ansprüche 1 bis 5,
**dadurch gekennzeichnet,**
**dass** einzelne Kammern (4 a, b, c, d) über eine Verbindungsleitung (7) mit einander verbunden sind.

7. Vorrichtung nach einem der Ansprüche 1 bis 6,
**dadurch gekennzeichnet,**
**dass** die Vorrichtung (4) auf einem Überzug (6) aufgebracht ist oder in Kombination mit einem Überzug (6) eingesetzt wird.

8. Vorrichtung nach einem der Ansprüche 1 bis 7,
**dadurch gekennzeichnet,**
**dass** die Vorrichtung (4) aus einer Kombination aus mindestens einer ringförmigen Manschette und/oder einer kappenartigen Manschette und/oder einem Ballon aufgebaut ist.

9. Verwendung einer Vorrichtung nach einem der Ansprüche 1 bis 8 zur Steuerung eines Endoskops.
